# FASCICULE DE BREVET EUROPEEN

(11) **EP 0 651 811 B1**
(45) Date de publication et mention de la délivrance du brevet: **16.11.2005**
(21) Numéro de dépôt: 93914819.3
(22) Date de dépôt: 06.07.1993
(51) Int. Cl.: C12N 15/81, C12N 15/62, C12N 15/14, C12N 1/21, C12N 15/12, C12N 15/19

(54) **PROMOTEUR DU GENE DE LA PROTEINE RIBOSOMIQUE RP28 DE K. LACTIS ET SON UTILISATION**
PROMOTOR VOM GEN DES K.LACTIS RP28 RIBOSOMALEN PROTEINS UND ANWENDUNG DAVON
K. LACTIS RP28 RIBOSOMAL PROTEIN GENE PROMOTER AND USE THEREOF

(30) Priorité: 08.07.1992 FR 9208429
(43) Date de publication de la demande: 10.05.1995
(73) Titulaire: Aventis Pharma S.A., 92160 Antony (FR)
(72) Inventeur: MENART, Sandrine, F-91940 Les Ulis (FR); BOLOTIN, Monique Résidence de Courcelles, F-91190 Gif-sur-Yvette (FR)
(74) Mandataire: Le Pennec, Magali
(86) Numéro de dépôt international: PCT/FR1993/000695
(87) Numéro de publication internationale: WO 1994/001570

(56) Documents cités:
- EP-A- 0 361 991
- JOURNAL OF BASIC MICROBIOLOGY vol. 28, no. 4, 1988, BERLIN, DE pages 211 - 220 CHEN, X.J. ET AL. 'A gene-cloning system for Kluyveromyces lactis and isolation of a chromosomal gene required for killer toxin production' cité dans la demande

### LISTE DE SEQUENCES

(1) INFORMATION GENERALE:
   (i) DEPOSANT:
      (A) NOM: RHONE-POULENC RORER S.A.
      (B) RUE: 20, avenue Raymond ARON
      (C) VILLE: ANTONY
      (E) PAYS: FRANCE
      (F) CODE POSTAL: 92165
   (ii) TITRE DE L' INVENTION: Promoteur de levure et son utilisation.
   (iii) NOMBRE DE SEQUENCES: 3
   (iv) FORME LISIBLE PAR ORDINATEUR:
      (A) TYPE DE SUPPORT: Tape
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
(2) INFORMATION POUR LA SEQ ID NO: 1:
   (i) CARACTERISTIQUES DE LA SEQUENCE:
      (A) LONGUEUR: 970 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (vi) ORIGINE:
      (A) ORGANISME: Kluyveromyces lactis
   (ix) CARACTERISTIQUE ADDITIONELLE:
      (A) NOM/CLE: CDS
      (B) EMPLACEMENT: 883..970
   (xi) DESCRIPTION DE LA SEQUENCE: SEQ ID NO: 1:
   AAGCTTTAAT GCGGTAGTTT ATCACAGTTA AATTGCTAAC GCAGTCAGGC ACCGTGTATG     60
   AAATCTAACA ATGCGCTCAT CGTCATCCTC GGCACCGTCA CCCTGGATGC TGTAGGCATA    120
   GGCTTGGTTA TGCCGGTACT GCCGGGCCTC TTGCGGGATA TCGTCCATTC CGACAGCATC    180
   GCCAGTCACT ATGGGTGCTG CTAGCGCTAT ATGCGTTGAT GCAATTTCTA TGCGCACCCG    240
   TTCTCGGAGC ACTGTCCGAC CGCTTTGGCC GCCGCCCAGT CCTGCTCGCT TCGCTACTTG    300
   GAGCCACTAT CGACTACGCG ATCATGGCGA CCACACCCTG TCCTGTGGAT CAATAGAAGG    360
   GTTCAACCAT TTCATCATTT TTGTTTCTGT ATTGATGTTG TGCGGAAGTA TATGTTATGT    420
   GATTTCCAGG TACCTCTCTG TAGGAGCCAA GTTTGTAAAA TTTTAGGTCT ACTGGCAGCA    480
   TAACTAAACG GAAACATATT CACACTTATA TCCAATATAT ATATGTACAT GATATAATTG     540
   ACAGATTTTA ACTGTTGATC GTCAGTCTGA TGTGATGACA CTGTCACTGG ACAGAAGCCG     600
   AACACAGGTC ACATGACTAA CATATGTTTT CTGGGACTGT TTGAGAATGT ACGGATGTAG     660
   TTTTTTCAAA ACTAGAAAAC CAACAATATA TTACCTAAAT AGGAATAAAG TTTTCAAAAC     720
   TCATCAACTC ACTGACATCG ACAAATTTCA CATCGTTACG AGTAGTGCAT ATATGAAGAA     780
   ACCATCTTTC AATACAGTTA ATCATACCAT TAAGCATATG TTCTAATGTA CTATTATCTC     840
   GATTAATTCT TGAAAGAACA CACACAATAA GCAAGTCTCA AA ATG GGT ATT GAT        894
                                                  Met Gly Ile Asp
                                                   1
   CAC ACT TCC AAG CAA CAC AAG AGA TCC AGT CAC AGA ACT GCT CCA AAG       942
   His Thr Ser Lys Gln His Lys Arg Ser Ser His Arg Thr Ala Pro Lys
     5                  10                  15                  20
   TCC GAC AAC GTC TAC TTG AAG TTG TTG G                                 970
   Ser Asp Asn Val Tyr Leu Lys Leu Leu
                   25
(2) INFORMATION POUR LA SEQ ID NO: 2:
   (i) CARACTERISTIQUES DE LA SEQUENCE :
      (A) LONGUEUR: 19 paires de bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (iii) ANTI-SENS: NON
   (xi) DESCRIPTION DE LA SEQUENCE: SEQ ID NO: 2:
   CTGTTTCATT TGAAGCGCG 19
(2) INFORMATION POUR LA SEQ ID NO: 3:
   (i) CARACTERISTIQUES DE LA SEQUENCE:
      (A) LONGUEUR: 549 paires de bases
      (B) TYPE: acide nucléique
      (C) NOMBRE DE BRINS: double
      (D) CONFIGURATION: linéaire
   (xi) DESCRIPTION DE LA SEQUENCE: SEQ ID NO: 3:
   ACGCGTGTCG ACGATCAATA GAAGGGTTCA ACCATTTCAT CATTTTTGTT TCTGTATTGA
   TGTTGTGCGG AAGTATATGT TATGTGATTT CCAGGTACCT CTCTGTAGGA GCCAAGTTTG
   TAAAATTTTA GGTCTACTGG CAGCATAACT AAACGGAAAC TATTCACACT TATATCCAAT
   ATATATATGT ACATGATATA ATTGACAGAT TTTAACTGTT GATCGTCAGT CTGATGTGAT
   GACACTGTCA CTGGACAGAA GCCGAACACA GGTCACATGA CTAACATATG TTTTCTGGGA
   CTGTTTGAGA ATGTACGGAT GTAGTTTTTT CAAAACTAGA AAACCAACAA TATATTACCT
   AAATAGGAAT AAAGTTTTCA AAACTCATCA ACTCACTGAC ATCGACAAAT TTCACATCGT
   TACGAGTAGT GCATATATGA AGAAACCATC TTTCAATACA GTTAATCATA CCATTAAGCA
   TATGTTCTAA TGTACTATTA TCTCGATTAA TTCTTGAAAG AACACACACA ATAAGCAAGT
   CTCAAGCTT

## Description

La présente invention concerne le domaine de la biologie moléculaire. Plus particulièrement, elle concerne une nouvelle séquence d'ADN présentant une activité de promoteur transcriptionnel, des vecteurs d'expression contenant cette séquence, et son utilisation pour la production de protéines recombinantes, et par exemple de protéines hétérologues. L'invention concerne aussi les cellules recombinées contenant cette séquence d'ADN.

Les progrès accomplis dans le domaine de la biologie moléculaire ont permis de modifier des microorganismes pour leur faire produire des protéines hétérologues. En particulier, de nombreuses études génétiques ont porté sur la bactérie *E.coli*. Toutefois, l'application industrielle de ces nouveaux modes de production est encore limitée, en particulier par les problèmes d'efficacité d'expression des gènes dans ces microorganismes recombinés. Aussi, dans le but d'augmenter les performances de ces systèmes de production, des recherches ont été effectuées afin d'isoler des promoteurs forts, permettant d'obtenir des niveaux élevés d'expression de protéines hétérologues. Chez *E.coli,* on peut citer en particulier les promoteurs des opérons tryptophane et lactose.

Plus récemment, chez la levure *S.cerevisiae,* des études ont porté sur des promoteurs dérivés de gènes impliqués dans la glycolyse. On peut citer notamment les travaux sur le promoteur du gène de la 3-phosphoglycérate kinase PGK (Dobson et al., Nucleic Acid Res. 10, 1982, 2625; Hitzeman et al., Nucleic Acid Research 1982, 7791), sur celui du gène de la glyceraldéhyde-3-phosphate deshydrogénase GAPDH (Holland et al., j.Biol.Chem. 254, 1979, 9839; Musti et al., Gene 25, 1983,133), sur celui du gène de l'alcool deshydrogénase 1 ADH1 (Bennentzen et al., J.Biol.Chem. 257, 1982, 3018; Denis et al., J.Biol.Chem. 25, 1983, 1165), sur celui du gène de l'enolase 1 ENO1 (Uemura et al., Gene 45, 1986, 65), sur celui du gène GAL1 /GAL10 (Johnston et Davis, Mol. Cell. Biol. 4 (1984) 1440) ou sur celui du gène CYC1 (Guarente et Ptashne, PNAS 78 (1981) 2199).

Récemment, des outils génétiques ont été développés afin de se servir de la levure *Kluyveromyces* comme cellule hôte pour la production de protéines recombinantes. La mise en évidence d'un plasmide de type 2-micron originaire de *K*. *drosophilarum* (plasmide pKD1 - EP 241435) a permis d'établir un système hôte/vecteur très efficace pour la production de protéines recombinantes (EP 361 991). Cependant, les promoteurs utilisés dans ce système n'ont pas été optimisés jusqu'à présent. En particulier, il s'agit essentiellement de promoteurs hétérologues, c'est-à-dire provenant d'autres microorganismes, tel que notamment *S.cerevisiae.* Cette situation peut engendrer différents inconvénients, et notamment limiter l'activité du promoteur à cause de l'absence de certains éléments de la machinerie transcriptionnelle (par exemple de trans-activateurs), présenter une certaine toxicité pour la cellule hôte due à une absence de régulation, ou affecter la stabilité du vecteur.

Dans ces conditions, le manque de promoteurs homologues forts chez *Kluyveromyces* constitue un facteur limitant dans l'exploitation industrielle de ce système d'expression.

La Demanderesse a maintenant identifié, cloné et séquence une région du génome de *Kluyveromyces lactis* présentant une activité de promoteur transcriptionnel (SEQ ID n° 1). Plus précisément, cette région correspond au promoteur du gène de la protéine ribosomique rp28 de *K*. *lactis* (Klrp28). Cette région, ou des dérivés ou fragments de celle-ci, peut être utilisée de manière très performante pour la production de protéines recombinantes chez les levures du genre *Kluyveromyces.* Il est entendu que cette séquence peut également être utilisée dans d'autres organismes hôtes.

Par ailleurs, un avantage de la région promotrice obtenue réside dans l'absence de répression par le glucose, permettant une utilisation dans des milieux de culture classiques et industriels.

Un objet de la présente invention réside donc dans une séquence d'ADN comprenant tout ou partie de la séquence SEQ ID n° 1 ou de son brin complémentaire, ou d'un dérivé de celles-ci, et possédant une activité de promoteur transcriptionnel.

Au sens de la présente invention, on entend par dérivé, toute séquence obtenue à partir de la séquence SEQ ID n° 1 par modification(s) de nature génétique et/ou chimique, conservant une activité de promoteur. Par modification de nature génétique et/ou chimique, on entend toute mutation, délétion, substitution, addition, et/ou modification d'un ou plusieurs nucléotides. De telles modifications peuvent être effectuées dans différents buts, et notamment celui de préparer des promoteurs portables, ou celui de préparer des promoteurs adaptés à l'expression dans un type particulier de vecteur ou d'hôte, celui de réduire la taille, d'augmenter l'activité de promoteur de la transcription, de générer des promoteurs inductibles, d'améliorer le niveau de régulation, ou encore de changer la nature de la régulation. De telles modifications peuvent être effectuées par exemple par mutagénèse *in vitro,* par introduction d'éléments additionnels de contrôle ou de séquences synthétiques, ou par des délétions ou des substitutions des éléments originels de contrôle.

Lorsqu'un dérivé tel que défini ci-dessus est réalisé, son activité de promoteur transcriptionnel peut être mise en évidence de plusieurs façons, et en particulier en plaçant sous le contrôle de la séquence étudiée, un gène reporteur dont l'expression est détectable. Toute autre technique connue de l'homme de l'art peut bien évidemment être utilisée à cet effet.

La séquence SEQ ID n°1 a été obtenue à partir d'une banque de fusion entre des fragments du génome de *K*. *lactis* 2359/152 et le gène lacZ de *E.coli* selon le protocole décrit dans les exemples. Il est entendu que l'homme du métier peut isoler cette région par hybridation au moyen d'une sonde comprenant tout ou partie de la séquence SEQ ID n° 1 ou de son brin complémentaire. Les dérivés selon l'invention peuvent ensuite être préparés à partir de cette séquence, comme indiqué dans les exemples.

Un autre objet de l'invention concerne un ADN recombinant comprenant une séquence d'ADN telle que définie ci-dessus.

Cet ADN recombinant peut contenir par exemple la séquence promotrice SEQ ID n° 1 ou un dérivé de celle-ci, dans laquelle est inséré un site de restriction, facilitant l'utilisation de cette séquence comme promoteur "portable".

Préférentiellement, cet ADN recombinant contient en outre un ou plusieurs gènes de structure. En particulier, il peut s'agir de gènes codant pour des protéines d'intérêt pharmaceutique ou agroalimentaire. A titre d'exemple, on peut citer les enzymes (tels que notamment la superoxide dismutase, la catalase, les amylases, les lipases, les amidases, la chymosine, etc), les dérivés sanguins (tels que la sérum-albumine, l'alpha- ou la béta-globine, le facteur VIII, le facteur IX, le facteur de von Willebrand, la fibronectine, l'alpha-1 antitrypsine, etc), l'insuline et ses variants, les lymphokines (telles que les interleukines, les interférons, les facteurs de stimulation des colonies [G-CSF, GM-CSF, M-CSF...], le TNF, le TRF, etc), les facteurs de croissance (tels que l'hormone de croissance, l'érythropoiétine, le FGF, l'EGF, le PDGF, le TGF, etc), les apolipoprotéines, des polypeptides antigéniques pour la réalisation de vaccins (hépatite, cytomégalovirus, Eppstein-Barr, herpes, etc), ou encore des fusions de polypeptides telles que notamment des fusions comportant une partie active fusionnée à une partie stabilisatrice (par exemple des fusions entre l'albumine ou des fragments d'albumine et le récepteur ou une partie d'un récepteur de virus [CD4, etc.]).

Encore plus préférentiellement, l'ADN recombinant contient également des signaux permettant la sécrétion du produit d'expression du ou desdits gènes de structure. Ces signaux peuvent correspondre aux signaux naturels de sécrétion de la protéine considérée, mais ils peuvent être d'une origine différente. En particulier des signaux de sécrétion dérivés de gènes de levure peuvent être utilisés, tels que ceux des gènes de la toxine killer (Stark et Boyd, EMBO J. 5 (1986) 1995) ou de la phéromone alpha (Kurjan et Herskowitz, Cell 30 (1982) 933; Brake et al., Yeast 4 (1988) S436).

Dans un mode de réalisation particulier de l'invention, l'ADN recombinant fait partie d'un plasmide d'expression, qui peut être à réplication autonome ou intégratif.

En particulier, des vecteurs à réplication autonome peuvent être obtenus en utilisant des séquences à réplication autonomes chez l'hôte choisi. Notamment, chez la levure, il peut s'agir d'origines de réplication dérivées de plasmides (pKD1, 2µ, etc), ou bien de séquences chromosomiques (ARS).

Les vecteurs intégratifs peuvent être obtenus notamment en utilisant des séquences homologues à certaines régions du génome de l'hôte, permettant, par recombinaison homologue, l'intégration du vecteur.

Un autre objet de l'invention concerne les cellules recombinées contenant une séquence d'ADN telle que définie ci-avant.

Avantageusement, les cellules sont choisies parmi les levures, et encore plus préférentiellement, parmi les levures du genre *Kluyveromyces.* Il est entendu cependant que l'invention couvre toutes les cellules recombinées dans lesquelles les régions promotrices de l'invention sont actives, qu'il s'agisse de cellules eucaryotes ou procaryotes.

Ainsi, parmi les cellules eucaryotes, on peut citer les cellules végétales, animales, les levures, ou les champignons. En particulier, s'agissant de levures, on peut citer les levures du genre *Saccharomyces, Pichia, Schwanniomyces,* ou *Hansenula.* S'agissant de cellules animales, on peut citer les cellules COS, CHO, C127, etc. Parmi les champignons susceptibles d'être utilisés dans la présente invention, on peut citer plus particulièrement Aspergillus ssp. ou *Trichoderma* ssp. Comme hôtes procaryotes, on peut utiliser les bactéries telles que *Escherichia coli,* ou celles appartenant aux genres *Corynebacterium, Bacillus,* ou *Streptomyces.*

L'activité de promoteur de la transcription des séquences de l'invention dans ces différents hôtes peut être vérifiée par exemple en introduisant dans la cellule hôte considérée un ADN recombinant comprenant, sous le contrôle de la séquence promotrice étudiée, un gène reporteur dont l'expression peut être mise en évidence dans l'hote considéré.

Les cellules recombinées de l'invention peuvent être obtenues par toute méthode permettant d'introduire un ADN étranger dans une cellule. Il peut s'agir notamment de transformation, électroporation, conjugaison, fusion de protoplastes, ou toute autre technique connue de l'homme de l'art. S'agissant de la transformation, différents protocoles ont été décrits dans l'art antérieur. En particulier, elle peut être réalisée en traitant les cellules entières en présence d'acétate de lithium et de polyéthylène glycol selon la technique décrite par Ito et al. (J. Bacteriol. 153 (1983) 163-168), ou en présence d'éthylène glycol et de diméthylsulfoxyde selon la technique de Durrens et al. (Curr. Genet. 18 (1990) 7). Un protocole alternatif a également été décrit dans la demande de brevet EP 361991. S'agissant d'électroporation, elle peut être réalisée selon Becker et Guarentte (in : Methods in Enzymology Vol194 (1991) 182).

Un autre objet de l'invention concerne l'utilisation d'une séquence telle que précédemment définie pour l'expression de gènes recombinés. Les séquences d'ADN selon l'invention peuvent en effet permettre une production à des niveaux élevés de protéines recombinantes.

Avantageusement, les séquences de l'invention peuvent être utilisées pour l'expression de gènes codant pour des protéines d'intérêt pharmaceutique ou agroalimentaire. A titre d'exemple, on peut citer les protéines énumérées précédemment.

La présente invention permet également la réalisation d'un procédé de production de protéines recombinantes, selon lequel on cultive une cellule recombinée telle que définie ci-avant et on récupère la protéine produite. A titre d'exemple de protéine, on peut citer les protéines énumérées précédemment.

Préférentiellement, le procédé de l'invention est applicable à la production de sérum-albumine humaine, ou un de ses variants moléculaires. On entend par variant moléculaire de l'albumine, les variants naturels résultant du polymorphisme de l'albumine, les formes tronquées, ou toute protéine hybride à base d'albumine.

D'autres avantages de la présente invention apparaîtront à la lecture des exemples qui suivent, qui doivent être considérés comme illustratifs et non limitatifs.

### LEGENDE DES FIGURES

- SEQ ID n°1 :: Séquence nucléotidique du fragment de 0,9 kb correspondant au promoteur Klrp28 de K. lactis.
- Figure 1 :: Préparation du transposon Mini Mu MudIIZK1.
- Figure 2 :: Carte de restriction du transposon Mini Mu MudIIZK1.
- Figure 3 :: Carte de restriction du clone 12C10.
- Figure 4:: Carte de restriction du fragment BamHI-HindIII de 1,75 kb portant le promoteur Klrp28.

### TECHNIQUES GENERALES DE CLONAGE

Les méthodes classiquement utilisées en biologie moléculaire telles que les extractions préparatives d'ADN plasmidique, la centrifugation d'ADN plasmidique en gradient de chlorure de césium, l'électrophorèse sur gels d'agarose ou d'acrylamide, la purification de fragments d'ADN par électroélution, les extraction de protéines au phénol ou au phénolchloroforme, la précipitation d'ADN en milieu salin par de l'éthanol ou de l'isopropanol, la transformation dans *Escherichia coli* etc, sont bien connues de l'homme de métier et sont abondament décrites dans la littérature [Maniatis T. et al., "Molecular Cloning, a Laboratory Manual", Cold Spring Harbor Laboratory, Cold Spring Harbor, N.Y., 1982; Ausubel F.M. et al. (eds), "Current Protocols in Molecular Biology", John Wiley & Sons, New York, 1987].

Les enzymes de restriction ont été fournies par New England Biolabs (Biolabs), ou Pharmacia et sont utilisées selon les recommandations des fournisseurs.

Les plasmides de type pBR322 et pUC sont d'origine commerciale (Bethesda Research Laboratories).

Pour les ligatures, les fragments d'ADN sont séparés selon leur taille par électrophorèse en gels d'agarose ou d'acrylamide, extraits au phénol ou par un mélange phénol/chloroforme, précipités à l'éthanol puis incubés en présence de l'ADN ligase du phage T4 (Boehringer) selon les recommandations du fournisseur.

Le remplissage des extrémités 5' proéminentes est effectué par le fragment de Klenow de l'ADN Polymérase 1 d'*E. coli* (Boehringer) selon les spécifications du fournisseur. La destruction des extrémités 3' proéminentes est effectuée en présence de l'ADN Polymérase du phage T4 (Biolabs) utilisée selon les recommandations du fabricant. La destruction des extrémités 5' proéminentes est effectuée par un traitement ménagé par la nudéase S1.

La mutagénèse dirigée *in vitro* par oligodéoxynucléotides synthétiques est effectuée selon la méthode développée par Taylor et al. [Nucleic Acids Res. 13 (1985) 8749-8764].

L'amplification enzymatique de fragments d'ADN par la technique dite de PCR [Polymérase-catalyzed Chain Reaction, Saiki R.K. et al., Science 230 (1985) 1350-1354; Mullis K.B. et Faloona F.A., Meth. Enzym. 155 (1987) 335-350] est effectuée en utilisant un "DNA thermal cycler" (Perkin Elmer Cetus) selon les spécifications du fabricant.

La vérification des séquences nucléotidiques est effectuée par la méthode développée par Sanger et al. [Proc. Natl. Acad. Sci. USA, 74 (1977) 5463-5467].

Les transformations de *K*. *lactis* sont effectuées par toute technique connue de l'homme de l'art, et dont un exemple est donné dans le texte.

Sauf indication contraire, les souches bactériennes utilisées sont *E.coli* DH1 (Hanahan D., J. Mol. Biol. 166 (1983) 557) ou *E.coli* JM109::(Mucts) (Daignan-fornier et Bolotin-Fukuhara, Gene 62 (1988) 45).

Les souches de levures utilisées appartiennent aux levures bourgeonnantes et plus particulièrement aux levures du genre *Kluyveromyces.* Les souche *K. lactis* 2359/152 et *K. lactis* SD6 ont été particulièrement utilisées.

Les souches de levures transformées par les plasmides sont cultivées en erlenmeyers ou en fermenteurs pilotes de 21 (SETRIC, France) à 28°C en milieu riche (YPD : 1 % extrait de levure, 2 % Bactopeptone, 2 % glucose; ou YPL : 1 % extrait de levure, 2 % Bactopeptone, 2 % lactose) sous agitation constante.

### EXEMPLES

### I - Isolement du promoteur Klrp28 de K. lactis.

La séquence SEQ ID n° 1 a été isolée à partir d'une banque de fusion entre des fragments du génome de *K. lactis* 2359/152 et le gène lacZ de *E.coli.* Cet exemple décrit en (A) la préparation de la banque de fusion, et en (B) la sélection et la caractérisation d'un clone de cette banque portant le promoteur du gène de la protéine ribosomique rp28 de *K. lactis.*

### A/ Préparation de la banque de fusion

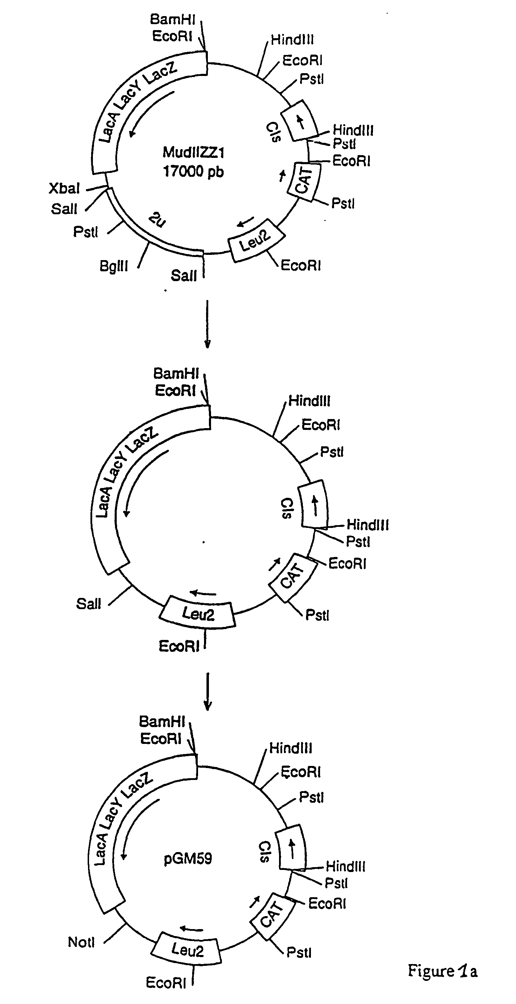

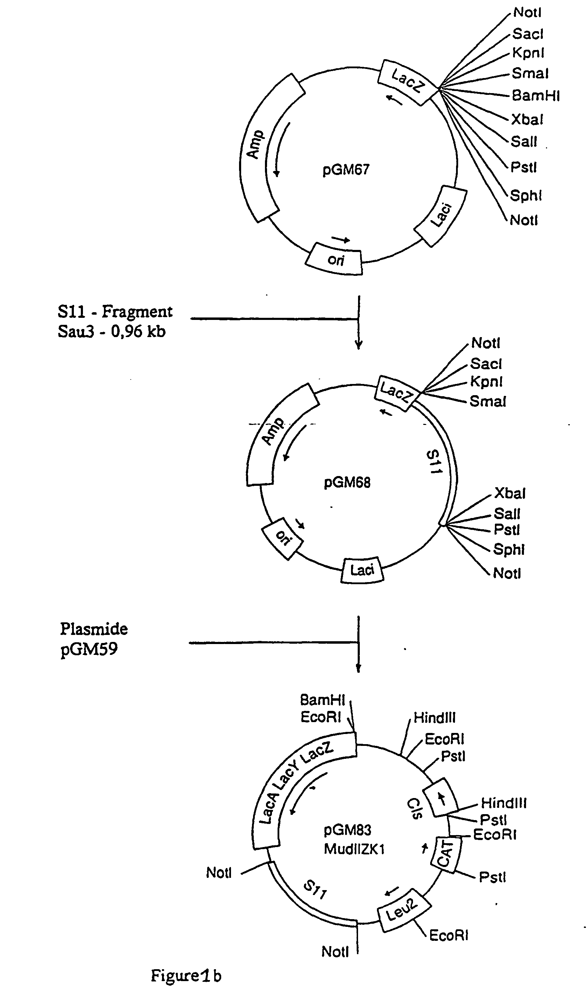

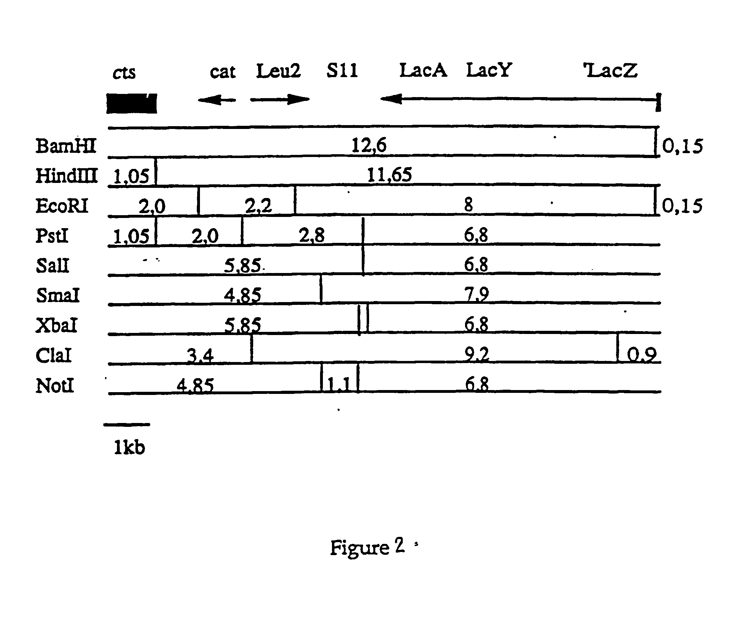

### A.1. Préparation du transposon Mini Mu MudIIZK1 (figures 1 et 2).

Le Mini Mu MudIIZK1 a été construit à partir du Mini Mu MudIIZZ1 décrit par Daignan-Fornier et Bolotin-Fukuhara (Gene 62 (1988) 45). Il a été obtenu en substituant l'origine de réplication du mini transposon MudIIZZ1 par une origine de réplication fonctionnelle dans *Kluyveromyces :* l'origine de réplication du plasmide pKD1 (EP231435).

### A.1.1. Construction d'une cassette portant l'origine de réplication du plasmide pKD1 (fragment S11).

Afin de faciliter les manipulations ultérieures, le fragment S11 (portant l'origine de réplication du plasmide pKD1) a été mis sous forme d'une cassette NotI. Pour cela, un dérivé du plasmide pUC18 a été construit dans lequel les sites externes du multisite de clonage (sites HindIII et EcoRI) ont été changés en sites Notl. Cela a été fait par digestion avec l'enzyme correspondante, action de l'enzyme de Klenow et ligature avec un oligonucléotide synthétique correspondant à un site NotI [oligo d(AGCGGCCGCT); Biolabs]. Le plasmide obtenu est désigné pGM67. Le fragment S11 de 960 pb obtenu par digestion par l'enzyme Sau3A du plasmide KEp6 (Chen et al, Nucl. Acids Res. 114 (1986) 4471) a ensuite été inséré au site compatible BamHI du plasmide pGM67. Le plasmide ainsi obtenu désigné pGM68 contient, sous forme d'une cassette NotI, le fragment S11.

### A.1.2. Suppression de l'origine de réplication 2µ du transposon MudIIZZ1.

Le plasmide pGM15 portant le mini Mu MudIIZZ1 (Daignan-Fornier et Bolotin-Fukuhara précitée) a été délété des régions 2µ par digestion au moyen de l'enzyme SalI. Le site SalI unique ainsi obtenu a ensuite été transformé en site NotI par ligature d'un oligonucléotide synthétique correspondant à un site NotI après action de l'enzyme de Klenow. Le plasmide résultant est appelé pGM59.

### A.1.3. Insertion du fragment S11

La cassette NotI portant l'origine de réplication du plasmide pKD1 (fragment S11), provenant du plasmide pUC18 modifié a ensuite été introduite au site NotI unique du plasmide pGM59.

Le plasmide obtenu, désigné pGM83, porte un mini Mu, appelé MudIIZK1 qui est adapté à la levure *Kluyveromyces lactis,* ainsi qu'une copie fonctionnelle du gène LEU2 de *S.cerevisiae* capable de complémenter une mutation leu2 chez *K.lactis* (Kämper et al, Curr. Genet. 19 (1991) 109). La carte de restriction du mini-mu MudIIZK1 est représentée sur la figure 2.

### A.2. Introduction du Mini Mu MudIIZK1 dans la souche E.coli portant le Mu helper JM109::(Mucts) : Obtention de la souche JM109::(Mucts)::(MudIIZK1).

La souche JM109::(Mucts) a été transformée par le plasmide pGM83 contenant le mini mu MudIIZK1 en présence de chlorure de calcium. Après transformation, la transposition a été induite par choc thermique selon la technique décrite par Castilho et al. (J. Bacteriol. 158 (1984) 488). Le lysat phagique obtenu après induction est ensuite utilisé pour surinfecter la souche JM109::(Mucts). La souche JM109::(Mucts) étant recA, l'ADN linéaire encapsidé par le phage ne peut se refermer pour donner un plasmide réplicatif. Les intégrants [souche JM109::(Mucts)::(MudIIZ1)] sont donc sélectionnés comme clones chloramphénicol résistants (Cm^{R}), ampicilline sensibles (Amp^{S}).

### A.3. Préparation de la banque génomique de K. lactis dans E.coli DH1

L'ADN de haut poids moléculaire a été préparé à partir de la souche *K. lactis* 2359/152, et digéré partiellement par l'enzyme Sau3A. Les fragments d'une taille de 4 à 8 kb ont été récupérés sur gel d'agarose LMP ("Low Melting Point", SEAKEM) et clonés dans le plasmide pBR322 linéarisé par BamHI et déphosphorylé par action de la phosphatase intestinale de veau (Biolabs). 35 pools de 1000 colonies dans *E.coli* DH1 ont ainsi été réalisés. Les 1000 colonies de chaque pool sont ampicilline-résistantes et tétracycline-sensibles, ce qui montre qu'elles ont toutes inséré un fragment d'ADN génomique de *K. lactis* dans pBR322.

### A.4. Préparation de la banque de fusion

### A.4.1. Introduction de la banque génomique de K.lactis dans la souche JM109::(Mucts)::(MudIIZK1).

L'ADN plasmidique de chaque pool réalisé dans DH1 est extrait (Maniatis). Cet ADN est ensuite utilisé pour transformer la souche JM109::(Mucts)::(MudlIZK1) en présence de chlorure de calcium. Pour être représentatif des 1000 colonies contenues dans chaque pool de la banque génomique, plus de 3000 clones par pool ont été récupérés dans la souche JM109::(Mucts)::(MudIIZK1) permettant la transduction.

### A.4.2. Transposition du Mini Mu MudIIZK1

La banque de fusion est réalisée par transposition extensive du Mini Mu MudIIZK1 sur les plasmides formant la banque d'ADN génomique de *K. lactis.* Les mini-muductions ont été faites selon le protocole décrit par Castilho et al. (J. Bacterioi. 158 (1984) 488) et les transductants ont été sélectionnés sur milieu sélectif LBAC (milieu LB (Gibco BRL) supplémenté avec 50 mg/l d'ampicilline et 30 mg/l de chloramphénicol), le marqueur Amp^{R} étant apporté par le plasmide, et le marqueur Cm^{R} par le mini-mu. Pour chaque pool, des transpositions sont faites en série, et entre 10 000 et 20 000 transductants sont récupérés par pool. L'ADN des transductants est ensuite extrait d'une préparation de 100 ml, purifié par précipitation au polyéthylène glycol (Maniatis et al, 1989) et resuspendu dans 100µl d'eau. Cet ADN a ensuite été utilisé pour transformer *K.lactis* et sélectionner des clones portant des promoteurs.

### B/ Isolement du promoteur Klrp28 de K.lactis

L'ADN de fusion préparé ci-dessus a été utilisé pour transformer, par électroporation, une souche réceptrice de *K.lactis.* Cette souche réceptrice, désignée SD6, porte les mutations leu2 (correspondant au marqueur de sélection du mini-mu MudIIZK1) et lac4-8. Cette dernière mutation empêche la souche de pousser sur un milieu contenant du lactose comme seule source de carbone, mais elle peut être complémentée par la surexpression du gène lacZ d'*E.coli* codant pour la β-galactosidase (Chen et al., J. Basic Microbiol. 28 (1988) 211). De ce fait, l'expression d'une protéine fusionnée à la β-galactosidase doit permettre la croissance de la souche SD6 sur lactose après transformation. Ce crible positif a été utilisé pour sélectionner rapidement des clones portant des promoteurs forts.

### B.1. Construction de la souche réceptrice K.lactis SD6.

La souche SD6 (Chen et al., Mol. Gen. Genet. 233 (1992) 97) a été obtenue par croisement de la souche *K.lactis* CXJ1-7A (a, lac4-8, ura3A, ade1-1, K1, K2, pKD1) (Chen et Fukuhara, Gene 69 (1988) 181) avec la souche AWJ-137 (leu2, trp1, homothallique) (Kämper et al, Curr. Genet. 19 (1991) 109), et sélection des spores ayant le génotype ADE⁺, uraA, leu2, lac4-8. Comme les spores obtenues n'étaient pas capables de régénérer après tranformation par protoplastes, un croisement retour a été fait avec la souche CXJ1-7A. Après sporulation en masse, les spores du génotype choisi ont été testées par transformation au chlorure de lithium avec le plasmide KEp6 selon une technique dérivée de celle décrite par Ito et al. (J. Bacteriol. 153 (1983) 163) (la concentration en LiCl est de 20 mM, soit 10 fois moins que celle utilisée par Ito pour *S.cerevisiae*)*.* La souche CXJ1-7A a servi de témoin de transformation.

La souche SD6, selectionnée sur ces critères, se transforme correctement : 1 à 3 . 10⁴ transformants par µg d'ADN ; et les transformants ont une stabilité satisfaisante : 30 à 40 % des colonies gardent le phénotype [Ura⁺] après 6 générations en milieu non sélectif.

### B.2. Isolement du promoteur Klrp28.

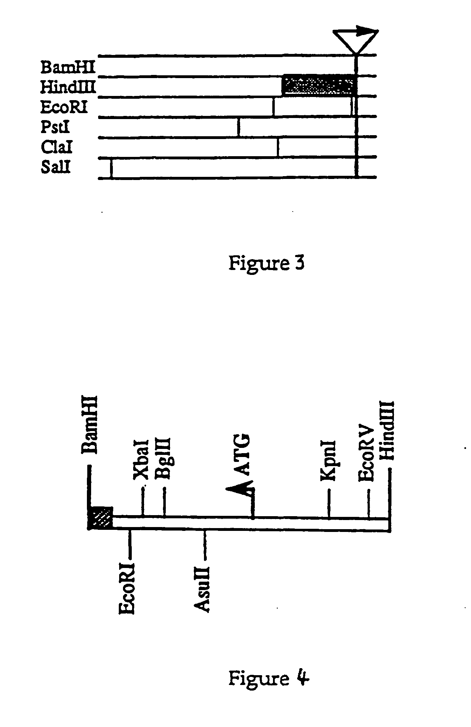

La souche SD6 a été transformée par électroporation selon Becker et Guarante (in Methods in Enzymology vol194 (1991) 182) (appareil Jouan; 2500 V/cm; 80-100 ng d'ADN/transformation) avec l'ADN de 11 pools de transductants obtenus en A.4.2. (correspondant à une banque de 11000 clones dans *E.coli*)*.* Après 5 heures de régénération en milieu YPD (extrait de levure 10 g/l; peptone 10 g/l; glucose 20 g/l), les cellules ont été étalées sur milieu minimum lactose. Les transformants capables de pousser sur lactose ont été restriés et, pour chaque clone, le plasmide a été extrait, amplifié dans *E.coli,* et, après vérification rapide de la carte de restriction du vecteur et du mini-mu, utilisé pour retransformer la levure SD6. Parmi les clones de *K. lactis* obtenus après retransformation, l'un d'entre-eux, le clone 12C10, a été étudié par restriction (voir figure 3) et par analyse de la séquence de la jonction entre la protéine de *K. lactis* et la β-galactosidase. Pour cela, la séquence de la jonction, à partir de l'extrémité lacZ du mini-mu (séquence double brin) a été déterminée par séquençage, au moyen de l'oligonucléotide suivant situé à -59 nucléotides de la jonction :

L'analyse de la séquence protéique déduite de la séquence nucléotidique ainsi obtenue par comparaison avec les séquences de banques de protéines d'autres levures ou eucaryotes (Genbank, MIPS, EMBL, etc), montre que la séquence portée par le clone 12C10 correspond au promoteur du gène de la protéine ribosomique rp28 de *K.lactis.* Le fragment BamHI-HindIII de 1,75 kb contenant la région en amont de la fusion a ensuite été sous-cloné dans le vecteur Bluescript KS+ (Stratagène), une carte de restriction a été faite (figure 4), et la séquence a été déterminée par délétions séquentielles sur 0,9 kb (SEQ ID n° 1). L'obtention d'éléments de séquence permet également à l'homme du métier de préparer des sondes spécifiques et de recloner la région promotrice de l'invention par hybridation selon les techniques classiques de biologie moléculaire.

### II - Transformation de Kluyveromyces.

Différentes techniques permettant l'introduction d'ADN dans la levure peuvent être utilisées.

Avantageusement, les différentes souches de *Kluyveromyces* utilisées ont été transformées en traitant les cellules entières en présence d'acétate de lithium et de polyéthylène glycol, selon la technique décrite par Ito et al. (J. Bacteriol. 153 (1983) 163-168). La technique de transformation décrite par Durrens et al. (Curr.Genet. 18 (1990) 7) utilisant l'éthylène glycol et le diméthylsulfoxyde a également été utilisée. Il est aussi possible de transformer les levures par électroporation, par exemple selon la méthode décrite par Karube et al. (FEBS Letters 182 (1985) 90).

Un protocole alternatif a encore été décrit en détail dans la demande EP 361991.

### III - Utilisation du promoteur de la figure 1 pour l'expression de gènes hétérologues.

L'activité de promoteur transcripdonnel de la région de *K. lactis* décrite sur la SEQ ID n° 1 a été mise en évidence lors même de son isolement, par sa capaciter à induire la complémentation de la mutation lac4-8 de la souche SD6. Cette capacité résulte en effet de l'expression du gène lacZ de *E.coli,* et démontre par là même la capacité d'expression de gènes hétérologues.

### IV - Construction d'un promoteur Klrp28 portable

Un promoteur portable est préparé par PCR, par insertion sur le fragment BamHI-HindIII de 1,75 kb d'un site de restriction HindIII en position +1 par rapport au codon ATG du gène Klrp28. et des sites de restriction MluI et SalI à 533 pb en amont (SEQ ID n° 3). Le produit de PCR est cloné dans le vecteur pCRII (Invitrogen) pour générer le plasmide pYG177, permettant de ressortir le promoteur par simple digestion MluI-HindIII, facilitant ainsi le clonage dans un vecteur d'expression.

Un vecteur d'expression de la sérum-albumine humaine est ensuite préparé à partir du plasmide pYG1018 comme suit : Le plasmide pYG1018 contient le gène prépro-albumine sous controle du promoteur LAC4. Il dérive du vecteur pYG1023 décrit dans la demande de brevet EP 402 212, par délétion du fragment BssHII-MluI portant le gène K1PGK. 5 µg des vecteurs pYG177 et pYG1018 sont digérés par 60 unités de HindIII et de MluI. Après migration sur gel d'agarose à 0,8 %, la bande correspondant au promoteur rp28 (environ 0,5 kb), la bande correspondant à la partie vecteur (environ 9 kb) et la bande correspondant au cDNA de l'albumine (environ 2 kb) sont électroéluées. Une ligation à 3 partenaires (suivant les recommandations de tampon et de températures définies par le fournisseur New England Biolabs) est ensuite effectuée avec 1 µl d'ADN promoteur, 1 µl d'ADN vectoriel, et 2 µl d'ADN albumine. Après transformation chez E.coli (Chung et al NAR 16 (1988) 3580), l'ADN plasmidique des transformants est préparé selon la technique de lyse alcaline au SDS de Birboim et Doly (NAR 6 (1979) 1513) modifiée par Ish-Horowicz et Burke (NAR 9 (1981) 2989). Après digestion enzymatique, le plasmide possédant le bon profil de restricion est isolé. Ce plasmide est désigné pYG183.

La souche K.lactis CBS 293.91 a été transformée par pYG183 dans les conditions décrites dans l'exemple II. La production d'albumine par plusieurs transformants est testée selon la technique décrite dans EP 361 991. La quantité d'albumine sécrétée par les transformants est similaire (50-100 mg/l).

## Revendications

1. Sequence d'ADN comprenant tout ou partie de la séquence SEQ ID n° 1, ladite partie possédant une activité de promoteur transcriptionnel.

2. ADN recombinant comprenant une séquence d'ADN selon la revendication. 1.

3. ADN recombinant selon la revendication 2 **caractérisé en ce qu'**il contient en outre un ou plusieurs gènes de structure.

4. ADN recombinant selon la revendication 3 **caractérisé en ce qu'**il contient également des signaux permettant la sécretion du produit d'expression du ou desdits gènes de structure.

5. ADN recombinant selon les revendications 3 et 4 **caractérisé en ce que** le ou les gènes de structure codent pour des protéines d'intérêt pharmaceutique ou agroalimentaire.

6. ADN recombinant selon la revendication 5 **caractérisé en ce que** le ou les gènes de structure codent pour des protéines choisies parmi les enzymes (tels que notamment la superoxide dismutase, la catalase, les amylases, les lipases, les amidases, la chymosine etc.), les dérivés sanguins (tels que la sérum-albumine, l'alpha- ou la béta-globine, le facteur VIII, le facteur IX, le facteur de von Willebrand, la fibronectine, l'alpha-1 antitrypsine etc.), l'insuline et ses variants, les lymphokines (telles que les interleukines, les interférons, les facteurs de stimulation des colonies (G-CSF, GM-CSF, M-CSF...), le TNF, le TRF etc.), les facteurs de croissance (tels que l'hormone de croissance, l'érythropoiétine, le FGF, l'EGF, le PDGF, le TGF etc.), les apolipoprotéines, des polypeptides antigéniques pour la réalisation de vaccins (hépatite, cytomégalovirus, Eppstein-Barr, herpes etc.), ou encore des fusions de polypeptides telles que notamment des fusions comportant une partie active fusionnée à une partie stabilisatrice (par exemple des fusions entre l'albumine ou des fragments d'albumine et le récepteur ou une partie d'un récepteur de virus (CD4, etc.)).

7. ADN recombinant selon l'une quelconque des revendications 2 à 6 **caractérisé en ce qu'**il fait partie d'un plasmide d'expression, qui peut être à réplication autonome ou intégratif.

8. Utilisation d'une séquence d'ADN selon l'une quelconque des revendications 1 à 7 pour l'expression de gènes recombinés.

9. Utilisation selon la revendication 8 pour l'expression de gènes codant pour des protéines d'intérêt pharmaceutique ou agroalimentaire.

10. Procédé de production de protéines recombinantes **caractérisé en ce que** l'on cultive une cellule recombinée comprenant une séquence d'ADN selon l'une des revendications 1 à 7 et on récupère les protéines produites.

11. Procédé selon la revendication 10, pour la production de protéines d'intérêt pharmaceutique ou agroalimentaire.

12. Procédé selon la revendication 10, dans lequel la protéine d'intérêt pharmaceutique ou agroalimentaire est choisie parmi les enzymes (tels que notamment la superoxide dismutase, la catalase, les amylases, les lipases, les amidases, la chymosine etc.), les dérivés sanguins (tels que la sérum-albumine, l'alpha- ou la béta-globine, le facteur VIII, le facteur IX, le facteur de von Willebrand, la fibronectine, l'alpha-1 antitrypsine etc.), l'insuline et ses variants, les lymphokines (telles que les interleukines, les interférons, les facteurs de stimulation des colonies (G-CSF, GM-CSF, M-CSF...), le TNF, le TRF etc.), les facteurs de croissance (tels que l'hormone de croissance, l'érythropoiétine, le FGF, l'EGF, le PDGF, le TGF etc.), les apolipoprotéines, des polypeptides antigéniques pour la réalisation de vaccins (hépatite, cytomégalovirus, Eppstein-Barr, herpes etc.), ou encore des fusions de polypeptides telles que notamment des fusions comportant une partie active fusionnée à une partie stabilisatrice (par exemple des fusions entre l'albumine ou des fragments d'albumine et le récepteur ou une partie d'un récepteur de virus (CD4, etc.)).

13. Procédé selon la revendication 10 **caractérisé en ce que** la protéine est préférentiellement la sérum-albumine humaine ou un de ses variants moléculaires.

## Patentansprüche

1. DNA-Sequenz, die die Sequenz SEQ ID NO: 1 ganz oder einen Teil davon umfasst, wobei der genannte Teil Transkriptionspromotor-Aktivität besitzt.

2. Rekombinante DNA, die eine DNA-Sequenz gemäß Anspruch 1 umfasst.

3. Rekombinante DNA nach Anspruch 2, **dadurch gekennzeichnet, dass** sie außerdem ein Strukturgen oder mehrere Strukturgene enthält.

4. Rekombinante DNA nach Anspruch 2, **dadurch gekennzeichnet, dass** sie auch Signale enthält, die die Sekretion des Expressionsproduktes des Strukturgens oder der Strukturgene ermöglichen.

5. Rekombinante DNA nach den Ansprüchen 3 und 4, **dadurch gekennzeichnet, dass** das Strukturgen oder die Strukturgene für Proteine von pharmazeutischem oder agroalimentärem Interesse codiert/codieren.

6. Rekombinante DNA nach Anspruch 5, **dadurch gekennzeichnet, dass** das Strukturgen oder die Strukturgene für Proteine, ausgewählt unter Enzymen (wie insbesondere Superoxiddismutase, Catalase, Amylasen, Lipasen, Amidasen, Chymosin usw.), Blutderivaten (wie z.B. Serumalbumin, alpha- oder beta-Globin, Faktor VIII, Faktor IX, von Willebrand-Faktor, Fibronectin, alpha-1-Antitrypsin usw.), Insulin und seinen Varianten, Lymphokinen (wie z.B. Interleukine, Interferone, Kolonien stimulierende Faktoren (G-CSF, GM-CSF, M-CSF ...), TNF, TRF usw.), Wachstumsfaktoren (wie z.B. Wachstumshormon, Erythropoietin, FGF, EGF, PDGF, TGF usw.) den Apolipoproteinen, antigenen Polypeptiden zur Herstellung von Vakzinen (Hepatit, Cytomegalovirus, Eppstein-Barr, Herpes usw.) oder auch Fusionen von Polypeptiden wie insbesondere Fusionen, die einen aktiven Teil fusioniert mit einem stabilisierenden Teil umfassen (z.B. Fusionen zwischen Albumin oder Albuminfragmenten und dem Virusrezeptor oder einem Teil eines Virusrezeptors (CD4 usw.)) codiert/codieren.

7. Rekombinante DNA nach einem der Ansprüche 2 bis 6, **dadurch gekennzeichnet, dass** sie zu einem Expressionsplasmid gehört, das zur autonomen oder integrierenden Replikation fähig ist.

8. Verwendung einer DNA-Sequenz nach einem der Ansprüche 1 bis 7 zur Expression von rekombinierten Genen.

9. Verwendung nach Anspruch 8 zur Expression von Genen, die für Proteine von pharmazeutischem oder agroalimentärem Interesse codieren.

10. Verfahren zur Herstellung von rekombinanten Proteinen, **dadurch gekennzeichnet, dass** man eine rekombinierte Zelle kultiviert, die eine DNA-Sequenz nach einem der Ansprüche 1 bis 7 umfasst, und man die produzierten Proteine isoliert.

11. Verfahren nach Anspruch 10 zur Herstellung von Proteinen von pharmazeutischem oder agroalimentärem Interesse.

12. Verfahren nach Anspruch 10, in dem das Protein von pharmazeutischem oder agroalimentärem Interesse ausgewählt wird aus Enzymen (wie insbesondere Superoxiddismutase, Catalase, Amylasen, Lipasen, Amidasen, Chymosin usw.), Blutderivaten (wie z.B. Serumalbumin, alpha- oder beta-Globin, Faktor VIII, Faktor IX, von Willebrand-Faktor, Fibronectin, alpha-1-Antitrypsin usw.), Insulin und seinen Varianten, Lymphokinen (wie Interleukine, Interferone, Kolonien stimulierende Faktoren (G-CSF, GM-CSF, M-CSF ...), TNF, TRF usw.), den Wachstumsfaktoren (wie Wachstumshormon, Erythropoietin, FGF, EGF, PDGF, TGF usw.), den Apolipoproteinen, antigenen Polypeptiden für die Herstellung von Vakzinen (Hepatitis, Cytomegalovirus, Eppstein-Barr, Herpes usw.) oder auch Fusionen von Polypeptiden wie insbesondere Fusionen, die einen aktiven Teil fusioniert an einen stabilisierenden Teil umfassen (z.B. Fusionen zwischen Albumin oder Albuminfragmenten und dem Virusrezeptor oder einem Teil eines Virusrezeptors (CD4, usw.)).

13. Verfahren nach Anspruch 10, **dadurch gekennzeichnet, dass** das Protein vorzugsweise humanes Serumalbumin oder eine seiner molekularen Varianten ist.

## Claims

1. DNA sequence comprising all or part of the sequence SEQ ID No. 1, the said part possessing a transcriptional promoter activity.

2. Recombinant DNA comprising a DNA sequence according to Claim 1.

3. Recombinant DNA according to Claim 2, **characterized in that** it contains, in addition, one or more structural genes.

4. Recombinant DNA according to Claim 3, **characterized in that** it also contains signals permitting the secretion of the product of expression of the said structural gene(s).

5. Recombinant DNA according to Claims 3 and 4, **characterized in that** the structural gene(s) encode proteins of interest in the pharmaceutical or food industry.

6. Recombinant DNA according to Claim 5, **characterized in that** the structural gene(s) encode proteins chosen from enzymes (such as, in particular, superoxide dismutase, catalase, amylases, lipases, amidases, chymosin, and the like), blood derivatives (such as serum albumin, alpha- or beta-globin, factor VIII, factor IX, von Willebrand's factor, fibronectin, alpha₁-antitrypsin, and the like), insulin and its variants, lymphokines (such as interleukins, interferons, colony stimulating factors [G-CSF, GM-CSF, M-CSF, etc.], TNF, TRF, and the like), growth factors (such as growth hormone, erythropoietin, FGF, EGF, PDGF, TGF, and the like), apolipoproteins, antigenic polypeptides for the production of vaccines (hepatitis, cytomegalovirus, Eppstein-Barr, herpes, and the like), or alternatively fusions of polypeptides such as, in particular, fusions containing an active portion fused to a stabilizing portion (e.g. fusions between albumin or albumin fragments and the virus receptor or a portion of a virus receptor [CD4, and the like]).

7. Recombinant DNA according to any one of Claims 2 to 6, **characterized in that** it forms part of an expression plasmid, which can be autonomously replicating or integrative.

8. Use of a DNA sequence according to any one of Claims 1 to 7 for the expression of recombinant genes.

9. Use according to Claim 8, for the expression of genes encoding proteins of interest in the pharmaceutical or food industry.

10. Process for producing recombinant proteins, **characterized in that** a recombinant cell comprising a DNA sequence according to one of Claims 1 to 7 is cultured and the proteins produced are recovered.

11. Process according to Claim 10 for the production of proteins of interest in the pharmaceutical or food industry.

12. Process according to Claim 10, in which the protein of interest in the pharmaceutical or food industry is chosen from enzymes (such as, in particular, superoxide dismutase, catalase, amylases, lipases, amidases, chymosin, and the like), blood derivatives (such as serum albumin, alpha- or beta-globin, factor VIII, factor IX, von Willebrand's factor, fibronectin, alpha₁-antitrypsin, and the like), insulin and its variants, lymphokines (such as interleukins, interferons, colony stimulating factors [G-CSF, GM-CSF, M-CSF, etc.], TNF, TRF, and the like), growth factors (such as growth hormone, erythropoietin, FGF, EGF, PDGF, TGF, and the like), apolipoproteins, antigenic polypeptides for the production of vaccines (hepatitis, cytomegalovirus, Eppstein-Barr, herpes, and the like), or alternatively fusions of polypeptides such as, in particular, fusions containing an active portion fused to a stabilizing portion (e.g. fusions between albumin or albumin fragments and the virus receptor or a portion of a virus receptor [CD4, and the like]).

13. Process according to Claim 10, **characterized in that** the protein is preferably human serum albumin or one of its molecular variants.
